Europäisches Patentamt

European Patent Office

Office européen des brevets

(19)

(11) Publication number: **0 005 891**
**B1**

(12) **EUROPEAN PATENT SPECIFICATION**

(45) Date of publication of patent specification: **04.01.84**

(51) Int. Cl.³: **G 01 N 33/48**

(21) Application number: **79200277.6**

(22) Date of filing: **05.06.79**

(54) Process for the preparation of a unit for the determination of residues of antibiotics and sulphas in biological liquids and such units.

(30) Priority: **05.06.78 NL 7806086**

(43) Date of publication of application:
**12.12.79 Bulletin 79/25**

(45) Publication of the grant of the patent:
**04.01.84 Bulletin 84/1**

(84) Designated Contracting States:
**BE CH DE FR GB IT LU NL SE**

(56) References cited:
**GB - A - 1 467 439**
**US - A - 3 126 325**

**CHEMICAL ABSTRACTS, Vol. 83, no. 21, 24
November 1975, abstract: 176783k, page 400,
COLUMBUS, Ohio, USA, J.L. VAN OS et al.:
"Diffusion test for the determination of antibiotic
residues in milk"**
**CHEMICAL ABSTRACTS, Vol. 79, no. 17, 29
October 1973, abstract 103722x, page 311,
COLUMBUS, OHIO, USA, R. GUDDING et al.:
"Detection of sulfonamides in milk"**

(73) Proprietor: **Gist - Brocades N.V.
Wateringseweg 1 P.O. Box 1
NL-2600 MA Delft (NL)**

(72) Inventor: **Beukers, Robert
Sijtwinde 159
NL-2631 GZ Nootdorp (NL)**
Inventor: **Van Os, Jan Lambert
Laan van Nieuw Oosteinde 113
NL-2274 EC Voorburg (NL)**

(74) Representative: **Van der Straaten, Jan Anthony
et al,
c/o GIST-BROCADES N.V. Patents and
Trademarks Department Wateringseweg 1 P.O.
Box 1
NL-2600 MA Delft (NL)**

Courier Press, Leamington Spa, England.

(56) References cited:

**JOURNAL OF DAIRY SCIENCE, Vol. 50, no. 9,
1967, MAMPAIGN, ILLINOIS, USA, J.M.
PALMER et al.: "Simple ultra-sensitive test for
detecting penicillin in milk"
Acta Vet. Scand. 17 (1976), pages 458-464
(Gudding)**

**The file contains technical information
submitted after the application was filed and not
included in this specification**

Process for the preparation of units for the determination of
residues of antibiotics and sulphas in biological liquids, and such units

The invention relates to a process for the preparation of units for the determination of residues of antibiotics and sulphas in biological liquids, such as milk, meat juice, serum and urine. The invention also relates to the units.

A similar process has been described by Gudding, Acta Vet. Scand. 17 (1976) pages 458 to 464, using plates with an agar medium in the manner as described by Galesloot et al, Netherlands Milk and Dairy Journal 16 (1962) pages 89 to 95, in which, however, the agar medium has been adapted to enable determinations of sulphas by the addition of trimethoprim (Merck Index 9th Ed. No. 9377). Sulphas are, generally speaking, compounds with a substituted or unsubstituted $SO_2NH_2$-group at the para site of a substituted or unsubstituted aniline nucleus, such as sulphamethoxazole [4-amino-N-(5-methyl-3-isoxazolyl) benzenesulphonamide]. In this process use is made of, inter alia, the thermophilic microorganism Bacillus stearothermophilus var. calidolactis, which is preferably incubated at about 60°C in order to avoid interferences by microorganisms present in the sample to be tested. In addition to the fact that fresh plates have to be prepared for each determination, the result of the test can be read not earlier than 6 hours after starting it.

The practice, however, needs a quicker test, using ready-for-use requirements, and giving a result within a few hours. In addition, the practice needs a test, which does not necessarily involve qualified laboratory personnel, and which may be carried out by, e.g. the truck driver transporting the milk from the farmer to the factory.

GB—A—1 467 439 describes a test starting indeed from ready-for-use requirements. That test gives a result with $1\frac{1}{2}$ to 4 hours, generally within 2 to 3 hours, and may be carried out by unqualified personnel, but the test is suitable for the determination of only antibiotics in biological liquids, such as milk, meat juice, serum and urine and shows too little sensitivity for the determination of sulphas.

According to the invention the test described in GB—A—1 467 439 is adapted for the determination of sulphas, and with the same test-duration of 2 to 3 hours.

Therefore the invention provides a process for the preparation of units for the determination of residues of antibiotics and sulphas in biological liquids, such as milk, meat juice, serum or urine, said units including an agar medium inoculated with spores of *Bacillus stearothermophilus var. calidolactis* and containing trimethoprim, characterized in that spores of *Bacillus Stearothermophilus var. calidolactis* (LMD 74.1) and trimethoprim [2,4-diamino-5-(3,4,5-trimethoxybenzyl)-1,3-pyrimidine] are introduced into an optionally buffered unmodified agar solution in concentrations of $10^5$ to $10^8$ spores per ml and 10 to 120 $\mu$g of trimethoprim per litre of agar medium, that optionally nutrients and an indicator suited for detecting growth of the microorganism, are added to the solution and the agar solution is then allowed to solidify in tubes, the process being carried out under such conditions that the spores stay alive but cannot germinate because of lack of nutrients and/or because of low temperature.

The units prepared according to the invention enable to obtain a result, within $1\frac{1}{2}$ to 4 hours, generally within 2 to 3 hours, whether a sample of biological liquid contains or does not contain an antibiotic or a sulpha in excess of a predetermined concentration. It has surprisingly been found that the test carried out with these units succeeds without the adaption of the medium, as reported by Gudding, so that the medium described in GB—A—1 467 439 may be used as such. It has also surprisingly been found that the time for reading the result is not necessarily extended. Thus, by choosing a suitable trimethoprim concentration and reading on acid formation or reduction, for instance according to the vertical diffusion test method of GB—A—1 467 439, the test time according to that method may be maintained. It is appreciated that Gudding used a trimethoprim concentration of 0.25 $\mu$g/ml, whereas, according to the invention, the trimethoprim concentration is lower, as indicated hereinbefore.

Trimethoprim appeared not or substantially not to influence the keepability of the spores. Furthermore, such a unit may be storable for more than a year.

Examples of units useful for the purpose of the invention are transparent tubes, single or in a set or combined to a block of translucent material provided with a number of holes shaped therein.

The nutrients necessary for the growth of the microorganism are preferably included in a tablet or in a disc of filter paper or anything like that. In a preferred embodiment of the invention the units as produced by the process described hereinbefore without the inclusion of nutrients are used in combination with nutrients in a tablet or filter paper disc adapted to be placed upon the agar medium before carrying out the determination. Nutrients, e.g. in a tablet, may also be included in the units beforehand, whereby preferably measures are taken to avoid moisture transport from the agar medium into the tablet. This may be done, e.g. by coating the tablet with a moisture-resistant layer, for example a wax, which coating must disappear during the test. A wax having a melting temperature of 35 to 55°C, preferably 40 to 45°C, is suitable for that purpose. The nutrients must contain at least an assimilable carbon source, e.g. glucose, an assimilable nitrogen source, e.g. peptone, and a source of growth factor and minerals, e.g. yeast extract. If the nutrients are included in the agar medium the unit should be stored at temperatures below those where the spores germinate (2 to 10°C).

The indicator used is an acid-base indicator for a pH of about 5.5, preferably bromocresol-purple or phenolred, or a redox indicator, preferably 2,3,5-triphenyltetrazolium chloride or Brilliant black.

The strain *Bacillus stearothermophilus var. calidolactis* from which the spores are used in the process of the invention, has been deposited at the Laboratory of Microbiology of the Technical University of Delft under number LMD 74.1, where the strain is available to the public.

This microorganism is very sensitive for penicilins. It is growing fast and shows the additional advantages of the optimal growing temperature being so high that other microorganisms generally do not grow, resulting in only a small chance of those microorganisms being interfering. The microorganism further shows a high sensitivity for other antibiotics.

The preparation of the spore-containing agar medium is further described in GB—A—1 467 439.

In the determination of residues of antibiotics and sulphas in biological liquids, such as milk, meat juice, serum and urine, using the units as produced by the process of the invention, a predetermined amount of the sample to be tested is placed in the unit and is left there or removed after a sufficiently long time, e.g. 15 to 30 minutes for the diffusion of the residues of antibiotics and sulphas, subsequently if necessary the nutrients are placed on the agar medium, and the contents of the unit are incubated at or near the optimal temperature for the microorganism during a predetermined period after which the indicator-colour is observed, indicating the presence or absence of an antibiotic and/or sulpha above a certain minimum concentration. The test is very simple to be caried out, so that qualified personnel is not necessary for the test. The determination may be done in $1\frac{1}{2}$ to 4 hours, preferably 2 to 3 hours, after starting the test, which is markedly shorter than for the method described by Gudding.

## Claims

1. Process for the preparation of units for the determination of residues of antibiotics and sulphas in biological liquids, said units including an agar medium inoculated with spores of *Bacillus Stearothermophilus var. calidolactis* and containing trimethoprim, characterized in that spores of *Bacillus Stearothermophilus var. calidolactis* (LMD 74.1) and trimethoprim [2,4-diamino-5-(3,4,5-trimethoxybenzyl)-1,3-pyrimidine] are introduced into an optionally buffered unmodified agar solution in con-

centrations of $10^5$ to $10^8$ spores per ml and 10 to 120 $\mu$g of trimethoprim per litre of agar medium, that optionally nutrients and an indicator suited for detecting growth of the microorganism, are added to the solution and the agar solution is then allowed to solidify in tubes, the process being carried out under such conditions that the spores stay alive but cannot germinate because of lack of nutrients and/or because of low temperature.

2. The units as produced by the process of claim 1 without the inclusion of nutrients, in combinaiton with nutrients in a tablet or filter paper disc adapted to be placed upon the agar medium before carrying out the determination.

## Revendications

1. Procédé de préparation de dispositifs pour la détermination de résidus d'antibiotiques et de sulfamidés dans des liquides biologiques, lesquels dispositifs comprennent un milieu à la gélose inoculé de spores de *Bacillus stearothermophilus var. calidolactis* et contenant du triméthoprime, caractérisé en ce que des spores de *Bacillus Stearothermophilus var. calidolactis* (LMD 74.1) et du triméthoprime [2,4-diamino-5-(3,4,5-triméthoxybenzyl)-1,3-pyrimidine] sont introduits dans une solution de gélose non modifiée, éventuellement tamponnée à des concentrations de $10^5$ à $10^8$ spores par ml et de 10 à 120 $\mu$g de triméthoprime par litre de milieu à la gélose, qu'éventuellement des agents nutritifs et un indicateur propre à la détection de la croissance du micro-organisme sont ajoutés à la solution et la solution de gélose est ensuite mise à solidifier dans des tubes, le procédé étant exécuté dans des conditions telles que les spores restent vivants, mais ne puissent germer en raison de l'absence d'agents nutritifs et/ou en raison d'une basse température.

2. Dispositifs préparés par le procédé suivant la revendication 1, sans apport d'agents nutritifs, en combinaison avec des agents nutritifs dans un comprimé ou un disque de papier-filtre propre à être posé sur le milieu à la gélose avant l'exécution de la détermination.

## Patentansprüche

1. Verfahren zur Herstellung von Einheiten für die Bestimmung von Rückständen von Antibiotika und Sulfonamiden in biologischen Flüssigkeiten, wobei die Einheiten einen mit Sporen von Bacillus Stearothermophilus var. calidolactis geimpften und Trimethoprim enthaltenden Agarnährboden aufweisen, dadurch gekennzeichnet, dass man Sporen von Bacillus Stearothermophilus var. calidolactis (LMD 74.1) und Trimethoprim [2,4-Diamino-5-(3,4,5-trimethoxybenzyl)-1,3-pyrimidin] in Konzentrationen von $10^5$ bis $10^8$ Sporen pro ml bzw. 10 bis 120 $\mu$g Trimethoprim pro Liter Agarnährboden in eine gegebenenfalls gepufferte unmodifizierte Agarlösung einbringt, dass man gegebenenfalls Nährstoffe und einen für den Nachweis des Wachstums des Mikroorganismus geeigneten Indikator zu der Lösung zusetzt und die Agarlösung dann in Röhrchen erstarren lässt, wobei man das Verfahren unter solchen Bedingungen ausführt, dass die Sporen am Leben bleiben, aber wegen Mangels an Nährstoffen und/oder wegen der niedrigen Temperatur nicht keimen können.

2. Einheiten, hergestellt gemäss dem Verfahren nach Anspruch 1 ohne Einschluss von Nährstoffen, in Kombination mit Nährstoffen in einer Tablette oder Filtrierpapierscheibe, die dafür geeignet sind, vor der Ausführung der Bestimmung auf den Agarnährboden gelegt zu werden.